(19) Europäisches Patentamt
European Patent Office
Office européen des brevets

(11) Veröffentlichungsnummer : **0 546 591 A2**

(12) # EUROPÄISCHE PATENTANMELDUNG

(21) Anmeldenummer : **92203021.8**

(51) Int. Cl.[5] : **C07J 1/00,** A61K 31/565

(22) Anmeldetag : **24.09.92**

(30) Priorität : **24.09.91 DE 4132182**

(43) Veröffentlichungstag der Anmeldung : **16.06.93 Patentblatt 93/24**

(84) Benannte Vertragsstaaten : **PT**

(71) Anmelder : **SCHERING AKTIENGESELLSCHAFT**
**Müllerstrasse 170/178**
**W-1000 Berlin 65 (DE)**

(72) Erfinder : **Bohlmann, Rolf, Dr.**
**Kühler Weg 6a**
**W-1000 Berlin 19 (DE)**
Erfinder : **Künzer, Hermann, Dr.**
**Kösliner Strasse 19**
**W-1000 Berlin 65 (DE)**
Erfinder : **Muhn-Seipoldy, Hans-Peter, Dr.**
**Markgrafenstrasse 61**
**W-1000 Berlin 28 (DE)**
Erfinder : **Nishino, Yukishige, Dr.**
**Lanzendorfer Weg 14**
**W-1000 Berlin 22 (DE)**
Erfinder : **Schneider, Martin, Dr.**
**Schluchseestrasse 6a**
**W-1000 Berlin 28 (DE)**

(54) **11-Beta-Substituierte 14,17-Ethanoestratriene, Verfahren zur Herstellung dieser Verbindungen sowie ihre Verwendung zur Herstellung von Arzneimitteln.**

(57) Die vorliegende Erfindung, betrifft 11β-substituierte 14,17-Ethanoestratriene der allgemeinen Formel I

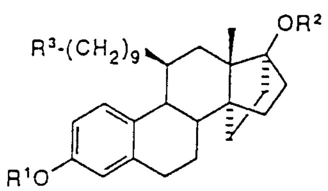

(I),

worin $R^1$ für ein Wasserstoffatom, eine $C_1$-$C_{12}$-Alkanoyl-, eine Benzoyl-, eine gerad- oder verzweigtkettige $C_1$-$C_{12}$-Alkyl-, eine $C_3$-$C_7$-Cycloalkyl- oder eine $C_4$-$C_8$-Alkylcycloalkylgruppe, $R^2$ für ein Wasserstoffatom oder eine $C_1$-$C_{12}$-Alkanoylogruppe und $R^3$ für eine Gruppierung

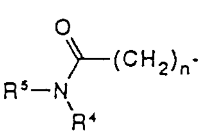 oder 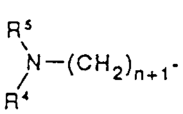 oder $R^4$-SO-

stehen, wobei n 0,1 oder 2 ist und $R^4$und $R^5$ unabhängig, voneinander ein Wasserstoffatom oder eine geradkettige oder verzweigte $C_1$-$C_8$-Alkylgruppe, die auch teilweise fluoriert vorliegen kann, bedeuten, ein Verfahren zu deren Herstellung, diese Verbindungen enthaltende pharmazeutische Präparate, sowie ihre Verwendung zur Herstellung, von Arzneimitteln.

EP 0 546 591 A2

Die vorliegende Erfindung betrifft 11β-substituierte 14,17-Ethanoestratriene der allgemeinen Formel I

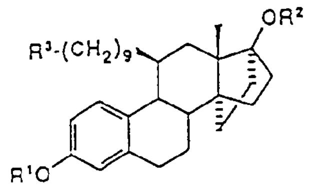

(I),

worin $R^1$ für ein Wasserstoffatom, eine $C_1$-$C_{12}$-Alkanoyl-, eine Benzoyl-, eine gerad- oder verzweigtkettige $C_1$-$C_{12}$-Alkyl-, eine $C_3$-$C_7$-Cycloalkyl- oder eine $C_4$-$C_8$-Alkylcycloalkylgruppe, $R^2$ für ein Wasserstoffatom oder eine $C_1$-$C_{12}$-Alkanoylgruppe und $R^3$ für eine Gruppierung

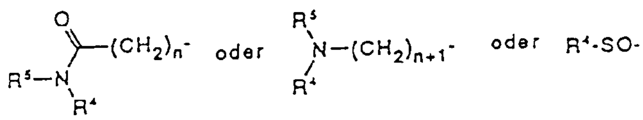

stehen, wobei n 0, 1 oder 2 ist und $R^4$ und $R^5$ unabhängig voneinander ein Wasserstoffatom oder eine geradkettige oder verzweigte $C_1$-$C_8$-Alkylgruppe, die auch teilweise fluoriert vorliegen kann, bedeuten, ein Verfahren zu deren Herstellung, diese Verbindungen enthaltende pharmazeutische Präparate, sowie ihre Verwendung zur Herstellung von Arzneimitteln.

Steht $R^1$ und/oder $R^2$ für eine Alkanoylgruppe, ist in erster Linie an eine Acetyl- oder Propionylgruppe gedacht; für $R^1$ in der Bedeutung einer Alkylgruppe kommen vor allem die Methyl- oder Ethylgruppe in Betracht. Als Cycloalkylgruppe für den Substituenten $R^1$ ist beispielsweise die Cyclopropyl-, Cyclopentyl- und Cyclohexylgruppe und als Alkylcycloalkylgruppe die Methylcyclopropyl- und Methylcyclopentylgruppe zu nennen.

Als teilweise fluorierte Alkylgruppen $R^4$ und $R^5$ kommen in erster Linie die Reste 2,2,3,3,4,4,4-Heptafluorbutyl- und 4,4,5,5,5-Pentafluorpentyl in Betracht.

Im Sinne der Erfindung besonders bevorzugte Verbindungen sind:
11-(14,17-Ethano-3,17-dihydroxyestra-1,3,5(10)-trien-11β-yl)-undecansäure-(N-methyl-N-*iso*propyl)-amid,
11-(14,17-Ethano-3,17-dihydroxyestra-1,3,5(10)-trien-11β-yl)-undecansäure-(N-methyl-N-2,2,3,3,4,4,4-heptafluorbutyl)-amid,
14,17-Ethano-11β-(11-N-methyl-N-*iso*propylaminoundecyl)-estra-1,3,5(10)-trien-3,17-diol,
9-(14,17-Ethano-3,17-dihydroxyestra-1,3,5(10)-trien-11β-yl)-nonyl-4,4,5,5,5-pentafluorpentyl-sulfoxid,
10-(14,17-Ethano-3,17-dihydroxyestra-1,3,5(10)-trien-11β-yl)-decansäure-(N-methyl-N-*iso*propyl)-amid,
12-(14,17-Ethano-3,17-dihydroxyestra-1,3,5(10)-trien-11β-yl)-dodecansäure-(N-methyl-N-*iso*propyl)-amid,
11-(3-Benzoyloxy-14,17-ethano-17-hydroxyestra-1,3,5(10)-trien-11β-yl)-undecansäure-(N-methyl-N-*iso*propyl)-amid,
11-(14,17-Ethano-17-hydroxy-3-methoxyestra-1,3,5(10)-trien-11β-yl)-undecansäure-(N-methyl-N-*iso*propyl)-amid.

Steroidale Antiestrogene, die im wesentlichen frei von einer estrogenen Restwirkung sind (reine Antiestrogene), gehen aus den EP-A 0 138 504 und EP 0 384 842 hervor. Aus der Vielzahl der in der EP-A 0 138 504 beschriebenen Substanzen ist insbesondere das 11-(3,17β-Dihydroxy-1,3,5(10)-estratrien-7α-yl)-undecansäure-(N-butyl-N-methyl)-amid hervorzuheben.

Eine Erhöhung der estrogenen Wirksamkeit gegenüber Estradiol bei peroraler Applikation durch Einführung einer 14,17-Ethanoüberbrückung wurde bei den in der internationalen Patentanmeldung WO 88/01 275 beschriebenen 14,17-Ethanoestratrienen beobachtet, die am C-7- und C-11-Atom je zwei Wasserstoffatome als Substituenten tragen.

Es wurde nunmehr gefunden, daß die erfindungsgemäßen Verbindungen der allgemeinen Formel I eine besonders hohe Affinität zum Estrogenrezeptor aufweisen und bei peroraler Applikation reine Antiestrogene mit sehr starker antiestrogener Wirkung sind.

Die erfindungsgemäßen Verbindungen eignen sich somit zur Therapie von estrogenabhängigen Erkrankungen, zum Beispiel anvulatorischer Infertilität, Prostatahyperplasie, Mammacarcinom, Endometriumcarcinom und Melanom.

Die tägliche Dosis zur Behandlung der genannten Krankheiten beträgt typischerweise 0,1 bis 25 mg/kg; beim Menschen entspricht dies einer täglichen Dosis von 5 bis 1250 mg. Eine Dosiseinheit enthält erfindungsgemäß 5 bis 500 mg einer oder mehrerer Verbindungen der allgemeinen Formel I.

Die erfindungsgemäßen Verbindungen sind zur Herstellung pharmazeutischer Zusammensetzungen und Zubereitungen geeignet. Die pharmazeutischen Zusammensetzungen beziehungsweise Arzneimittel enthalten als Wirkstoff einen oder mehrere der erfindungsgemäßen Verbindungen, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können.

Als derartige Träger- und Hilfsstoffe kommen zum Beispiel solche infrage, die in folgenden Literaturstellen als Hilfsstoffe für Pharmazie, Kosmetik und angrenzende Gebiete empfohlen beziehungsweise angegeben sind: Ullmans Encyklopädie der technischen Chemie, Band 4 (1953), Seite 1 bis 39; Journal of Pharmaceutical Sciences, Band 52 (1963), Seite 918 ff., H. v. Czetsch-Lindenwald, Hilfsstoffe für Pharmazie und angrenzende Gebiete; Pharm. Ind., Heft 2, 1961, Seite 72 u. ff.: Dr. H P. Fiedler, Lexikon der Hilfsstoffe für Pharmazie, Kosmetik und angrenzende Gebiete, Cantor KG. Aulendorf in Württemberg 1971.

Die Verbindungen können oral oder parenteral, beispielsweise intraperitoneal, intramuskulär, subkutan oder perkutan verabreicht werden. Die Verbindungen können auch in das Gewebe implantiert werden. Die zu verabreichende Menge der Verbidung schwankt innerhalb eines weiten Bereichs und kann jede wirksame Menge abdecken. In Abhängigkeit des zu behandelnden Zustands und der Art der Verabreichung kann die Menge der verabreichten Verbidung 0,01 - 100 mg/kg Körpergewicht, vorzugsweise 0,1 - 20 mg/kg Körpergewicht, je Tag betragen.

Zur oralen Verabreichung kommen Kapseln, Pillen, Tabletten, Dragees usw. infrage. Die Dosierungseinheiten können neben dem Wirkstoff einen pharmazeutisch verträglichen Träger, wie zum Beispiel Stärke, Zucker, Sorbit, Gelatine, Gleitmittel, Kieselsäure, Talkum usw., enthalten.

Zur parenteralen Verabreichung können die Wirkstoffe in einem physiologisch verträglichen Verdünnungsmittel gelöst oder suspendiert sein. Als Verdünnungsmittel werden sehr häufig Öle mit oder ohne Zusatz eines Lösungsvermittlers, eines oberflächenaktiven Mittels, eines Suspendier- oder Emulgiermittels verwendet. Beispiele für verwendete Öle sind Olivenöl, Erdnußöl, Baumwollsamenöl, Sojabohnenöl, Rizinusöl und Sesamöl.

Die Verbindungen lassen sich auch in Form einer Depotinjektion oder eines Implantatpräparats anwenden, die so formuliert sein können, daß eine verzögerte Wirkstoff-Freigabe ermöglicht wird.

Implantate können als inerte Materialien zum Beispiel biolobisch abbaubare Polymere enthalten oder synthetische Silikone wie zum Beispiel Silikonkautschuk. Die Wirkstoffe können außerdem zur perkutanen Applikation zum Beispiel in ein Pflaster eingearbeitet werden.

Die Verbindungen der allgemeinen Formel I werden erfindungsgemäß hergestellt, indem eine Verbindung der allgemeinen Formel II

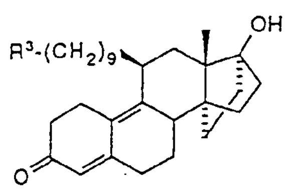

(II),

worin $R^3$ die in Formel I angegebenen Bedeutungen

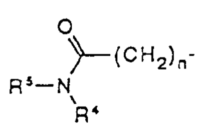 oder 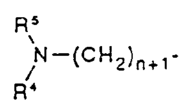 oder $R^4$-SO-

hat, mit einem Säurehalogenid/Säureanhydrid-Gemisch

$$R^{1,2}X/(R^{1,2})_2O \qquad (III),$$

worin $R^{1,2}$ eine $C_1$-$C_{12}$-Alkanoylgruppe sowie X ein Chlor- oder Bromatom bedeuten, zur entsprechenden 1,3,5(10)-Trien-3-hydroxy-Verbindung der allgemeinen Formel IV

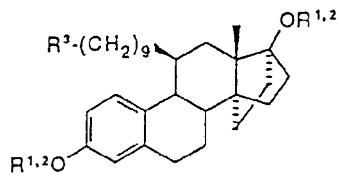

(IV),

aromatisiert und diese anschließend gegebenenfalls partiell oder vollständig verseift, gegebenenfalls partiell in 3-Position oder vollständig in 3- und 17-Position mit einem Säurehalogenid oder -anhydrid der allgemeinen Formel V

$$R^{1'}X \text{ bzw. } R^{1'}_2O \quad (V),$$

worin $R^{1'}$ eine $C_1$-$C_{12}$-Alkanoyl- oder Benzoylgruppe sowie X ein Chlor- oder Bromatom bedeuten, verestert oder partiell in 3-Position mit einem Alkylhalogenid der allgemeinen Formel VI

$$R^{1''}Y \quad (VI),$$

worin $R^{1''}$ eine gerad- oder verzweigtkettige $C_1$-$C_{12}$-Alkyl-, eine $C_3$-$C_7$-Cycloalkyl- oder eine $C_4$-$C_8$-Alkylcycloalkylgruppe sowie Y ein Chlor-, Brom- oder Iodatom bedeuten, verethert und gegebenenfalls in 17-Position mit einem Säurehalogenid- oder -anhydrid der allgemeinen Formel VII

$$R^{2'}X \text{ bzw. } R^{2'}_2O \quad (VII),$$

worin $R^{2'}$ eine $C_1$-$C_{12}$-Alkanoylgruppe sowie X ein Chlor- oder Bromatom bedeuten, verestert und wenn $R^3$ für den Carbonsäureamidrest

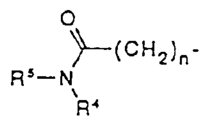

steht gewünschtenfalls dessen Ketogruppe vollständig reduziert wird.

Die Aromatisierung erfolgt mit einem Säurehalogenid/Säureanhydrid. Die partielle Veresterung oder Veretherung der 3-Hydroxygruppe bzw. die vollständige Veresterung der 3- und 17-Hydroxygruppe erfolgt nach gängigen Methoden. Die vollständige Reduktion der Ketogruppe des Carbonsäureamids kann mit Lithiumaluminiumhydrid oder ähnlichen Reduktionsmitteln nach ebenfalls üblichen Verfahren erfolgen.

Die nachfolgenden Beispiele dienen der Erläuterung der Erfindung. Innerhalb der Beispiele ist auch die Darstellung der benötigten Verbindungen der allgemeinen Formel II sowie erstmals die Darstellung von 4,4,5,5,5-Pentafluorpentanol sowie dessen Umsetzung zu 4,4,5,5,5-Pentafluorpentylmercaptan beschrieben. Weitere homologe Fluoralkanole, die zur Synthese weiterer erfindungsgemäßer Verbindungen, worin $R^4$ eine teilweise fluorierte Alkylgruppe darstellt, sind durch analoge Vorgehensweise erhältlich.

3,3-(2,2-Dimethyltrimethylendioxy)-5α,10α-epoxy-14,17-ethano-estr-9(11)-en-17-ol (Scholz, S. et. al., Liebigs Ann. Chem., (1989), S. 151 (13b)) wird über die 9(11)-Doppelbindung unter Kupfer-Katalyse in 11-Stellung mit einem Grignardreagenz der Formel

$$MgX\text{-}(CH_2)_9\text{-}(CH_2)_{n+1}\text{-}O\text{-}L,$$

worin X ein Chlor-, Brom- oder Iodatom, L eine Hydroxyschutzgruppe wie etwa die Tertiär-butyl-dimethylsilylgruppe und n 0, 1 oder 2 ist bzw. $MgX$-$(CH_2)_9$-$S$-$R^4$, worin X die obenstehende und $R^4$ die in Formel I angegebene Bedeutung haben, verknüpft.

Anschließend wird die 4,9-Dien-3-on-Struktur durch Behandlung des Grignard-Additionsproduktes im mäßig sauren Medium (beispielsweise in einem Gemisch aus Tetrahydrofuran und halbkonzentrierter Essigsäure) etabliert, wobei auch die gegebenenfalls vorhandene endständige Hydroxyschutzgruppe L abgespalten wird. Die ω-Hydroxygruppe kann dann gewünschtenfalls nach gängigen Verfahren zur entsprechenden Carbonsäure oxidiert werden (z.B. mit Jones'Reagenz). Die ω-Hydroxygruppe bzw. Carboxylgruppe wird abschließend mit einem Amin $H$-$NR^4R^5$, worin $R^4$ und $R^5$ die in Formel I angegebene Bedeutung haben, zu einer Verbidung der allgemeinen Formel II mit endständiger Amin- oder Amidgruppe im 11β-Alkylsubstituenten umgesetzt.

Im Falle der endständigen Funktion -S(O)-$R^4$ muß dieses Sulfid zum Erhalt einer Verbindung der allgemeinen Formel noch zum entsprechenden Sulfoxid -S-$R^4$, beispielsweise mit Natriumperiodat, aufoxidiert werden.

Beispiel 1

11-(14,17-Ethano-3,17-dihydroxyestra-1,3,5(10)-trien-11β-yl)-undecansäure-(N-methyl-N-*iso*propyl)-amid

a) 11-Bromundecyl*tertiär*butyldimethylsilylether

100 g 11-Bromundecanol (Fluka) werden in 240 ml Tetrahydrofuran gelöst und bei 25 °C mit 58 g Imidazol sowie 74,3 g *tertiär*Butyldimethylsilylchlorid in 80 ml Tetrahydrofuran versetzt und 2 h bei 25 °C gerührt. Zur Aufarbeitung gibt man 700 ml Diethylether hinzu, filtriert das ausgefallene Hydrochlorid ab,engt im Vakuum zur Trockne ein und chromatographiert an Kieselgel mit Hexan/Toluol. Man erhält 136,9 g 11-Bromundecyl*tertiär*butyldimethylsilylether als Öl.

b) 14,17-Ethano-11β-(11-hydroxyundecyl)-17-hydroxyestra-4,9-dien-3-on

7,2 g Magnsiumspäne werden in 70 ml Tetrahydrofuran vorgelegt und mit einer Lösung von 108 g 11-Bromundecyl*tertiär*butyldimethylsilylether in 140 ml Tetrahydrofuran innerhalb von 1,5 h versetzt. Nach 1 h bei 40 °C wird auf 0 °C abgekühlt, mit 1,3 g Kupfer(I)chlorid versetzt, 0,5 h bei 0 °C gerührt, eine Lösung von 10 g 3,3-(2,2-Dimethyltrimethylendioxy)-5α,10α-epoxy-14,17-ethanoestr-9(11)-en-17-ol (Scholz, S. et al. Liebigs Ann. Chem., (1989), S. 151 (13b)) in 80 ml Tetrahydrofuran zugetropft und 0,5 h bei 0 °C weitergerührt. Dann wird eine gesättigte Ammoniumchloridlösung zugegeben, 0,25 h bei 0 °C gerührt, mit Essigester verdünnt, mit Natriumchloridlösung gewaschen und im Vakuum zur Trockne eingeengt. Man erhält 14,2 g rohes 11β-(11-(Dimethyl*tertiär*butylsilyloxy)undecyl)-3,3-(2,2-dimethyltrimethylendioxy)-14,17-ethanoestr-9-en-5α,17β-diol. Das rohe 11β-(11-(Dimethyl*tertiär*butylsilyloxy)-undecyl)-3,3-(2,2-dimethyltrimethylendioxy)-14,17-ethanoestr-9-en-5α,17β-diol wird in 70 ml Tetrahydrofuran mit 80 ml Eisessig und 40 ml Wasser 1,5 h bei 50 °C Badtemperatur gerührt. Dann wird mit Essigester verdünnt, vier mal mit Natriumhydrogencarbonat und Kochsalzlösung gewaschen, über Natriumsulfat getrocknet, im Vakuum zur Trockne eingeengt und an Kieselgel mit Hexan/Essigester chromatographiert. Man erhält 4,8 g 14,17-Ethano-11β-(11-hydroxyundecyl)-17-hydroxyestra-4,9-dien-3-on,
$[\alpha]_D^{22}$ = -121,1°, als Öl.

d) 11-(14,17-Ethano-17-hydroxy-3-oxoestra-4,9-dien-11β-yl)-undecansäure-(N-methyl-N-*iso*propyl)-amid

1,0 g 14,17-Ethano-11β-(11-hydroxyundecyl)-17-hydroxyestra-4,9-dien-3-on werden in 17 ml Aceton bei 0 °C langsam mit 1,0 ml Jones-Reagens versetzt und 1 h gerührt. Zur Aufarbeitung werden 1,5 ml 2-Propanol zugegeben, im Vacuum zur Trockne eingeengt, 0,1 n Salzsäure zugegeben, viermal mit Dichlormethan extrahiert, mit Kochsalzlösung gewaschen, über Natriumsulfat getrocknet und im Vakuum zur Trockne eingeengt. Man erhält 860 mg rohe 11-(14,17-Ethano-17-hydroxyestra-4,9-dien-3-on-11β-yl)-undecansäure als Schaum. Die rohe 11-(14,17-Ethano-17-hydroxyestra-4,9-dien-3-on-11β-yl)-undecansäure wird in 15 ml Dichlormethan bei -10 °C mit 0,22 ml N-Methylmorpholin und 0,25 ml Chlorameisensäure*iso*butylester 0,5 h gerührt. Anschließend werden 0,27 ml N-Methyl-N-*iso*propylamin langsam zugetropft und 1 h bei 25 °C gerührt. Zur Aufarbeitung wird mit Natriumhydrogencarbonatlösung versetzt, mit Dichlormethan verdünnt, mit Wasser und Kochsalzlösung gewaschen, über Natriumsulfat getrocknet, im Vakuum zur Trockne eingeengt und an Kieselgel mit Dichlormethan/Aceton chromatographiert. Man erhält 655 mg 11-(14,17-Ethano-17-hydroxy-3-oxoestra-4,9-dien-11β-yl)-undecansäure-(N-methyl-N-*iso*propyl)-amid, $[\alpha]_D^{22}$ = -106.1°, als Öl.

e) 11-(14,17-Ethano-3,17-dihydroxyestra-1,3,5(10)-trien-11β-yl)-undecansäure-(N-methyl-N-*iso*propyl)-amid

500 mg 11-(14,17-Ethano-17-hydroxy-3-oxoestra-4,9-dien-11β-yl)-undecansäure-(N-methyl-N-*iso*propyl)-amid in 7 ml Dichlormethan werden bei 0 °C mit einer Lösung von 0,26 ml Acetylbromid in 0,51 ml Acetanhydrid langsam versetzt und 16 h bei 25 °C gerührt. Dann wird mit Dichlormethan verdünnt, mit Natriumhydrogencarbonatlösung, Wasser und Kochsalzlösung gewaschen, über Natriumsulfat getrocknet und im Vakuum zur Trockne eingeengt. Man erhält rohes 11-(3,17-Diacetoxy-14,17-ethanoestra-1,3,5(10)-trien-11β-yl)-undecansäure-(N-methyl-N-*iso*propyl)-amid als Öl.
Dieses Rohprodukt wird in 10 ml einer 1 normalen methanolischen Kaliumhydroxydlösung 1 h bei 25 °C gerührt. Dann wird mit 1n Salzsäure neutralisiert, im Vacuum eingeengt, auf Wasser gegeben, viermal mit Dichlormethan extrahiert, mit Kochsalzlösung gewaschen, über Natriumsulfat getrocknet, im Vakuum zur Trockne eingeengt und an Kieselgel mit Dichlormethan/Aceton chromatographiert. Man erhält reines 11-(14,17-Ethano-3,17-dihydroxyestra-1,3,5(10)-trien-11β-yl)-undecansäure-(N-methyl-N-*iso*propyl)-amid, $[\alpha]_D^{22}$ = +54,8°, als farblose Kristalle vom Schmelzpunkt 126-8°C.

Beispiel 2

11-(14,17-Ethano-3,17-dihydroxyestra-1,3,5(10)-trien-11β-yl)-undecansäure-(N-methyl-N-2,2,3,3,4,4,4-heptafluorbutyl)-amid

a) 11-(14,17-Ethano-17-hydroxy-3-oxoestra-4,9-dien-11β-yl)-undecansäure-(N-methyl-N-2,2,3,3,4,4,4-heptafluorbutyl)-amid

860 mg rohe 11-(14,17-Ethano-17-hydroxyestra-4,9-dien-3-on-11β-yl)-undecansäure werden in 15 ml Dichlormethan bei -10 °C mit 0,22 ml N-Methylmorpholin und 0,25 ml Chlorameisensäure*iso*butylester 0,5 h gerührt. Anschließend werden 0,27 ml N-Methyl-N-2,2,3,3,4,4,4-heptafluorbutylamin langsam zugetropft und 1 h bei 25 °C gerührt. Zur Aufarbeitung wird mit Natriumhydrogencarbonatlösung versetzt, mit Dichlormethan verdünnt, mit Wasser und Kochsalzlösung gewaschen, über Natriumsulfat getrocknet, im Vakuum zur Trockne eingeengt und an Kieselgel mit Dichlormethan/Aceton chromatographiert. Man erhält 581 mg 11-(14,17-Ethano-17-hydroxy-3-oxoestra-4,9-dien-11β-yl)-undecansäure-(N-methyl-N-2,2,3,3,4,4,4-heptafluorbutyl)-amid, $[\alpha]_D^{22}$ = -119,2°, als Öl.

b) 11-(14,17-Ethano-3,17-dihydroxyestra-1,3,5(10)-trien-11β-yl)-undecansäure-(N-methyl-N-2,2,3,3,4,4,4-heptafluorbutyl)-amid

500 mg 11-(14,17-Ethano-17-hydroxy-3-oxoestra-4,9-dien-11β-yl)-undecansäure-(N-methyl-N-2,2,3,3,4,4,4-heptafluorbutyl)-amid in 7 ml Dichlormethan werden bei 0 °C mit einer Lösung von 0,26 ml Acetylbromid in 0,51 ml Acetanhydrid langsam versetzt und 16 h bei 25 °C gerührt. Dann wird mit Dichlormethan verdünnt, mit Natriumhydrogencarbonatlösung, Wasser und Kochsalzlösung gewaschen, über Natriumsulfat getrocknet und im Vakuum zur Trockne eingeengt. Man erhält rohes 11-(3,17-Diacetoxy-14,17-ethanoestra-1,3,5(10)-trien-11β-yl)-undecansäure-(N-methyl-N-2,2,3,3,4,4,4-heptafluorbutyl)-amid als Öl.
Dieses rohe Amid wird in 10 ml einer 1 normalen methanolischen Kaliumhydroxydlösung 1 h bei 25 °C gerührt. Dann wird mit 1n Salzsäure neutralisiert, im Vacuum eingeengt, auf Wasser gegeben, viermal mit Dichlormethan extrahiert, mit Kochsalzlösung gewaschen, über Natriumsulfat getrocknet, im Vakuum zur Trockne eingeengt und an Kieselgel mit Dichlormethan/Aceton chromatographiert. Man erhält reines 11-(14,17-Ethano-3,17-dihydroxyestra-1,3,5(10)-trien-11β-yl)-undecansäure-(N-methyl-N-2,2,3,3,4,4,4-heptafluorbutyl)-amid, $[\alpha]_D^{22}$ = +67.1°, als farbloses Öl.

Beispiel 3

14,17-Ethano-11β-(11-N-methyl-N-*iso*propylaminoundecyl)-estra-1,3,5(10)-trien-3,17-diol

a) 14,17-Ethano-17-hydroxy-11β-(11-N-methyl-N-*iso*propylaminoundecyl)-estra-4,9-dien-3-on

1,0 g 14,17-Ethano-17-hydroxy-11β-(11-hydroxyundecyl)-estra-4,9-dien-3-on werden in 17 ml Dichlormethan bei -10 °C mit 0,22 ml N-Methylmorpholin und 0,25 ml Chlorameisensäure*iso*butylester 0,5 h gerührt. Anschließend werden 0,27 ml N-Methyl-N-*iso*propylamin langsam zugetropft und 1 h bei 25 °C gerührt. Zur Aufarbeitung wird mit Natriumhydrogencarbonatlösung versetzt, mit Dichlormethan verdünnt, mit Wasser und Kochsalzlösung gewaschen, über Natriumsulfat getrocknet, im Vakuum zur Trockne eingeengt und an Kieselgel mit Dichlormethan/Aceton chromatographiert. Man erhält 702 mg 14,17-Ethano-17-hydroxy-11β-(11-N-methyl-N-*iso*propylaminoundecyl)-estra-4,9-dien-3-on, $[\alpha]_D^{22}$ = -55.3°, als Öl.

b) 14,17-Ethano-11β-(11-N-methyl-N-*iso*propylaminoundecyl)-estra-1,3,5(10)-trien-3,17-diol

500 mg 14,17-Ethano-17-hydroxy-11β-(11-N-methyl-N-*iso*propylaminoundecyl)-estra-4,9-dien-3-on in 7 ml Dichlormethan werden bei 0 °C mit einer Lösung von 0,26 ml Acetylbromid in 0,51 ml Acetanhydrid langsam versetzt und 16 h bei 25 °C gerührt. Dann wird mit Dichlormethan verdünnt, mit Natriumhydrogencarbonatlösung, Wasser und Kochsalzlösung gewaschen, über Natriumsulfat getrocknet und im Vakuum zur Trockne eingeengt. Man erhält rohes 3,17-Diacetoxy-14,17-ethano-11β-(11-N-methyl-N-*iso*propylaminoundecyl)-estra-

1,3,5(10)-trien als Öl.
Dieses Rohprodukt wird in 10 ml einer 1 normalen methanolischen Kaliumhydroxydlösung 1 h bei 25 °C gerührt. Dann wird mit 1n Salzsäure neutralisiert, im Vacuum eingeengt, auf Wasser gegeben, viermal mit Dichlormethan extrahiert, mit Kochsalzlösung gewaschen, über Natriumsulfat getrocknet, im Vakuum zur Trockne eingeengt und an Kieselgel mit Dichlormethan/Aceton chromatographiert. Man erhält reines 14,17-Ethano-11β-(11-N-methyl-N-*iso*propylaminoundecyl)-estra-1,3,5(10)-trien-3,17-diol, $[\alpha]_D^{22}$ = +39,4°, als Schaum.

Beispiel 4

9-(14,17-Ethano-3,17-dihydroxyestra-1,3,5(10)-trien-11β-yl)-nonyl-4,4,5,5,5-pentafluorpentyl-sulfoxid

a) 4,4,5,5,5-Pentafluorpentanol

Eine Lösung von 25 g 4,4,5,5,5-Pentafluorpent-2-en-1-ol (Kitazume, T. and Ishikawa, N. J. Am. Chem. Soc., (1985), S. 5186) in 100 ml Essigester wird mit 100 mg Platindioxid unter einer Wasserstoffatmosphäre 1 Stunde geschüttelt. Dann wird der Katalysator abfiltriert und fraktionierend destilliert. Bei 133-135 °C erhält man 22 g 4,4,5,5,5-Pentafluorpentanol mit $^1$H-NMR ($CDCl_3$) δ: 1.86 (m, H2), 2.18(m, H1) und 3.75 (t, J=6.1 Hz, H1).

b) 4,4,5,5,5-Pentafluorpentylmercaptan

65 ml Azodicarbonsäureethylester werden mit einer Lösung von 107 g Triphenylphosphin in 800 ml Tetrahydrofuran 0.5 Stunden gerührt dann werden 30 ml Thioessigsäure sowie 20 g 4,4,5,5,5-Pentafluorpentanol in 100 ml Tetrahydrofuran langsam zugesetzt und 1 Stunde bei 0 °C sowie über Nacht bei 25 °C gerührt und unter vermindertem Druck fraktionierend destilliert. Das so erhaltene 4,4,5,5,5-Pentafluorpentyl-1-thioacetat wird mit 100 ml einer 2N-Natriumhydroxidlösung 3 Stunden bei 100 °C gerührt, abgekühlt mit 2N-Salzsäure auf pH 4 gebracht, mit Diethylether extrahiert, mit Kochsalzlösung gewaschen, über Natriumsulfat getrocknet und im Vakuum eingeengt. Man erhält 15 g rohes 4,4,5,5,5-Pentafluorpentylmercaptan als Öl.

c) 9-Brom-nonyl-4,4,5,5,5-pentafluorpentyl-sulfid

30 ml Azodicarbonsäureethylester werden mit einer Lösung von 50 g Triphenylphosphin in 400 ml Tetrahydrofuran 0.5 Stunden gerührt dann werden 10 g rohes 4,4,5,5,5-Pentafluorpentylmercaptan sowie 12 g 9-Brom-nonanol in 48 ml Tetrahydrofuran langsam zugesetzt und 1 Stunde bei 0 °C sowie über Nacht bei 25°C gerührt. Zur Aufarbeitung wird im Vakuum eingeengt und an Kieselgel mit Hexan/Essiester chromatographiert Man erhält 14 g 9-Brom-nonyl-4,4,5,5,5-pentafluorpentyl-sulfid als schwach gelbes Öl.

d) 9-(14,17-Ethano-17-hydroxy-3-oxoestra-4,9-dien-11β-yl)-nonyl-4,4,5,5,5-pentafluorpentyl-sulfoxid

0,6 g Magnesiumspäne werden in 6 ml Tetrahydrofuran vorgelegt und mit einer Lösung von 10 g 9-Bromnonyl-4,4,5,5,5-pentafluorpentyl-sulfid in 14 ml Tetrahydrofuran innerhalb von 1,5 g versetzt. Nach 1 h bei 40 °C wird auf 0 °C abgekühlt, mit 0,13 G Kupfer(I)chlorid versetzt, 0,5 h bei 0 °C gerührt, eine Lösung von 1,0 g 3,3-(2,2-Dimethyltrimethylendioxy)-5α,10α-epoxy-14,17-ethanoestr-9(11)-en-5α,17β-diol (Scholz, S. et al. Liebigs Ann. Chem., (1989), S. 151 (13b)) in 8 ml Tetrahydrofuran zugetropft und 0,5 h bei 0 °C weitergerührt. Dann wird mit eine gesättigte Ammoniumchloridlösung zugegeben, 0,25 h bei 0 °C gerührt, mit Essigester verdünnt, mit Natriumchloridlösung gewaschen und im Vakuum zur Trockne eingeengt. Man erhält 1,3 g rohes 9-(3,3-(2,2-Dimethyltrimethylendioxy)-14,17-ethano-5,17-dihydroxyestr-9-en-11β-yl)-nonyl-4,4,5,5,5-pentafluorpentylsulfid. Das rohe Sulfid wird in 7 ml Tetrahydrofuran mit 8 ml Eisessig und 4 ml Wasser 1,5 h bei 50 °C Badtemperatur gerührt. Dann wird mit Essigester verdünnt, vier mal mit Natriumhydrogencarbonat und Kochsalzlösung gewaschen, über Natriumsulfat getrocknet, im Vakuum zur Trockne eingeengt, in 15 ml Methanol gelöst, mit 440 mg Natriumperiodat behandelt, mit Essigester verdünnt, vier mal mit Natriumhydrogencarbonat und Kochsalzlösung gewaschen, über Natriumsulfat getrocknet, im Vakuum zur Trockne eingeengt und an Kieselgel mit Hexan/Essigester chromatographiert. Man erhält 985 mg 9-(14,17-Ethano-17-hydroxy-3-oxoestra-4,9-dien-11β-yl)-nonyl-4,4,5,5,5-pentafluorpentyl-sulfoxid, $[\alpha]_D^{22}$ = -75,3°, als Öl.

e) 9-(14,17-Ethano-3,17-dihydroxyestra-1,3,5(10)-trien-11β-yl)-nonyl-4,4,5,5,5-pentafluorpentyl-sulfoxid

500 mg 9-(14,17-Ethano-17-hydroxyestra-4,9-dien-3-on-11β-yl)-nonyl-4,4,5,5,5-pentafluorpentyl-sulfoxid in 7 ml Dichlormethan werden bei 0 °C mit einer Lösung von 0,26 ml Acetylbromid in 0,51 ml Acetanhydrid langsam versetzt und 2 h bei 25 °C gerührt. Dann wird mit Dichlormethan verdünnt, mit Natriumhydrogencarbonatlösung, Wasser und Kochsalzlösung gewaschen, über Natriumsulfat getrocknet und im Vakuum zur Trockne eingeengt. Man erhält rohes 9-(3,17-Diacetoxy-14,17-ethanoestra-1,3,5(10)-trien-11β-yl)-nonyl-4,4,5,5,5-pentafluorpentyl-sulfoxid als Öl.
Dieses Rohprodukt wird in 10 ml einer 1 normalen methanolischen Kaliumhydroxydlösung 1 h bei 25 °C gerührt. Dann wird mit 1n Salzsäure neutralisiert, im Vacuum eingeengt, auf Wasser gegeben, viermal mit Dichlormethan extrahiert, mit Kochsalzlösung gewaschen, über Natriumsulfat getrocknet, im Vakuum zur Trockne eingeengt und an Kieselgel mit Dichlormethan/Aceton chromatographiert. Man erhält reines 9-(14,17-Ethano-3,17-dihydroxyestra-1,3,5(10)-trien-11β-yl)-nonyl-4,4,5,5,5-pentafluorpentyl-sulfoxid, $[\alpha]_D^{22}$ = +43.2°, als farbloses Öl.

Beispiel 5

10-(14,17-Ethano-3,17-hydroxyestra-1,3,5(10)-trien-11β-yl)-decansäure-(N-methyl-N-*iso*propyl)-amid

Nach der Methode aus Beispiel 1 wird durch Verwendung von 10-Bromdecyl*tertiär*butyldimethylsilylether statt 11-Bromundecyl*tertiär*butyldimethylsilylether das 11-(14,17-Ethano-3,17-dihydroxyestra-1,3,5(10)-trien-11β-yl)-decansäure-(N-methyl-N-*iso*propyl)-amid als farblose Kristalle vom Schmelzpunkt 112-4 °C erhalten.

Beispiel 6

12-(14,17-Ethano-3,17-dihydroxyestra-1,3,5(10)-trien-11β-yl)-dodecansäure-(N-methyl-N-*iso*propyl)-amid

Nach der Methode aus Beispiel 1 wird durch Verwendung von 12-Bromdodecyl*tertiär*butyldimethylsilylether statt 11-Bromundecyl*tertiär*butyldimethylsilylether das 11-(14,17-Ethano-3,17-dihydroxyestra-1,3,5 (10)-trien-11β-yl)-decansäure-(N-methyl-N-*iso*propyl)-amid als farblose Kristalle vom Schmelzpunkt 107-9 °C erhalten.

Beispiel 7

11-(3-Benzoyloxy-14,17-ethano-17-hydroxyestra-1,3,5 (10)-trien-11β-yl)-undecansäure-(N-methyl-N-*iso*propyl)-amid

1,0 g 11-(14,17-Ethano-3, 17-dihydroxyestra-1,3,5(10)-trien-11β-yl)-undecansäure-(N-methyl-N-*iso*propyl)-amid werden in 11,3 ml Aceton bei 0 °C mit 3,2 ml 0,1 n-Natronlauge und 0,3 ml Benzoylchlorid versetzt und 0,5 h bei 0 °C nachgerührt. Zur Aufarbeitung wird auf Natriumhydrogencarbonat gegeben, dreimal mit Essigester extrahiert, mit Kochsalzlösung gewaschen, über Natriumsulfat getrocknet, im Vakuum zur Trockne eingeengt und an Kieselgel mit Hexan/Essigester chromatographiert. Man erhält 1,0 g 11-(3-Benzoyloxy-14,17-ethano-17-hydroxyestra-1,3,5 (10)-trien-11β-yl)-undecansäure-(N-methyl-N-*iso*propyl)-amid, $[\alpha]_D^{22}$ = +42,1°, als Schaum.

Beispiel 8

11-(14,17-Ethano-17-hydroxy-3-methoxyestra-1,3,5(10)-trien-11β-yl)-undecansäure-(N-methyl-N-*iso*propyl)-amid

1,0 g 11-(14,17-Ethano-3,17-hydroxyestra-1,3,5(10)-trien-11β-yl)-undecansäure-(N-methyl-N-*iso*propyl)-amid werden in 10 ml Acteon mit 3 g Kaliumcarbonat und 0,3 ml Methyliodid versetzt und 5 h bei 50 °C nachgerührt. Zur Aufarbeitung wird auf Natriumhydrogencarbonatlösung gegeben, dreimal mit Essigester extrahiert, mit Kochsalzlösung gewaschen, über Natriumsulfat getrocknet, im Vakuum zur Trockne eingeengt und an Kieselgel mit Hexan/Essigester chromatographiert. Man erhält 1,0 g 11-(14,17-Ethano-17-hydroxy-3-methoxyestra-1,3,5(10)-trien-11β-yl)-undecansäure-(N-methyl-N-*iso*propyl)-amid, $[\alpha]_D^{22}$ = +56,8°, als Schaum.

Die Verbindung des Beispiels 4 kann erfindungsgemäß auch wie nachstehend beschrieben hergestellt

werden:
9-(14,17-Ethano-3,17-dihydroxyestra-1,3,5(10)-trien-11β-yl)-nonyl-4,4,5,5,5-pentafluorpentyl-sulfoxid

a) 4,4,5,5,5-Pentafluorpentanol

Eine Lösung von 25 g 4,4,5,5,5-Pentafluorpent-2-en-1-ol (Kitazume, T. and Ishikawa, N. J. Am. Chem. Soc., (1985), S. 5186) in 100 ml Essigester wird mit 100 mg Platindioxid unter einer Wasserstoffatmosphäre 1 Stunde geschüttelt. Dann wird der Katalysator abfiltriert und fraktionierend destilliert. Bei 133-135 °C erhält man 22 g 4,4,5,5,5-Pentafluorpentanol mit $^1$H-NMR ($CDCl_3$) δ: 1.86 (m, H2), 2. 18 (m, H1) und 3.75 (t, J=6.1 Hz, H1).

b) Thioessigsäure-S-(4,4,5,5,5-pentafluorpentyl)-ester

Eine Lösung von 17.3 g 4,4,5,5,5-Pentafluorpentanol in 40 ml Pyridin wird bei 0 °C mit 21 g Tosylchlorid versetzt und 3 Stunden bei 0 °C gerührt. Zur Aufarbeitung wird die Reaktionsmischung auf 2 normale Schwefelsäure gegeben, mit Diethylether extrahiert mit Wasser neutralgewaschen, über Natriumsulfat getrocknet und i.Vak. eingeengt. Man erhält 32.7 g rohen Toluol-4-sulfonsäure-4,4,5,5,5-pentafluorpentylester als Öl. Dieser wird in 300 ml Aceton gelöst und mit 23 g Kaliumthioacetat 18 Stunden bei 100 °C Badtemperatur rückfluxiert. Dann wird mit Wasser versetzt, mit Diethylether extrahiert, mit Wasser gewaschen, über Natriumsulfat getrocknet, i.Vak. eingeengt und desilliert. Bei 173 - 177 °C erhält man 15 g Thioessigsäure-S-(4,4,5,5,5-pentafluorpentyl)-ester.

c) 14,17-Ethano-11β-(11-hydroxynonyl)-17-hydroxyestra-4,9-dien-3-on

8,5 g Magnsiumspäne werden in 85 ml Tetrahydrofuran vorgelegt und mit einer Lösung von 125 g 9-Bromnonyl*tertiär*butyldimethylsilylether in 150 ml Tetrahydrofuran innerhalb von 1,5 h versetzt. Nach 1 h bei 40 °C wird auf 0 °C abgekühlt, mit 1,5 g Kupfer(I)chlorid versetzt, 0,5,h bei 0 °C gerührt, eine Lösung von 33 g 3,3-(2,2-Dimethyltrimethylendioxy)-5α,10α-epoxy-14,17-ethanoestr-9(11)-en-17-ol (Scholz, S. et al. Liebigs Ann. Chem., (1989), S. 151 (13b)) in 150 ml Tetrahydrofuran zugetropft und 0,5 h bei 0 °C weitergerührt. Dann wird eine gesättigte Ammoniumchloridlösung zugegeben, 0,25 h bei 0 °C gerührt, mit Essigester verdünnt, mit Natriumchloridlösung gewaschen und im Vakuum zur Trockne eingeengt. Man erhält 49,2 g rohes 11β-(9-(Dimethyl*tertiär*butylsilyloxy)-nonyl)-3,3-(2,2-dimethyltrimethylendioxy)-14,17-ethanoestr-9-en-5α,17β-diol. Das rohe 11β-(11-(Dimethyl*tertiär*butylsilyloxy)-nonyl)-3,3-(2,2-dimethyltrimethylendioxy)-14,17-ethanoestr-9-en-5α,17β-diol wird in 180 ml Tetrahydrofuran mit 206 ml Eisessig und 103 ml Wasser 2,5 h bei 50 °C Badtemperatur gerührt. Dann wird mit Essigester verdünnt, vier mal mit Natriumhydrogencarbonat und Kochsalzlösung gewaschen, über Natriumsulfat getrocknet, im Vakuum zur Trockne eigeengt und an Kieselgel mit Hexan/Essigester chromatographiert. Man erhält 17.8 g 14,17-Ethano-11β-(11-hydroxynonyl)-17-hydroxyestra-4,9-dien-3-on, [α]22,D = -116.5°, Schaum.

d) 17-Acetoxy-11β-(9-acetoxynonyl)-14,17-ethanoestra-1,3,5(10)-trien-3-ol

Zu einer Lösung von 4.8 g 14,17-Ethano-11β-(11-hydroxynonyl)-17-hydroxyestra-4,9-dien-3-on in 40 ml Pyridin werden 20 ml Acetanhydrid und 400 mg Dimethylaminopyridin gegeben und 4 Stunden bei Raumtemperatur gerührt. Dann wird die Reaktionslösung auf eine Salzsaure Mischung von Eiswasser/Kochsalz gebeben, mit Essigester extrahiert, neutralgewaschen, über Natriumsulfat getrocknet und i.Vak. eingeengt. Man erhält 4.8 g rohes 17-Acetoxy-11β-(9-acetoxynonyl)-14,17-ethanoestra-4,9-dien-3-on, das in 75 ml Ethanol mit 1,5 g 10 % Palladium auf Kohle 21 Stunden bei 100 °C Badtemperatur gerührt wird. Dann wird über Celite filtriert, i. Vak. eingeengt und an Kieselgel mit Hexan/Essigester chromatographiert Man erhält 1,2 g reines 17-Acetoxy-11β-(9-acetoxynonyl)-14,17-ethanoestra-1,3,5( 10)-trien-3-ol.

e) 3-Benzoyloxy-14,17-ethano-11β-(9-tosyloxynonyl)-estra-1,3,5(10)-trien-17-ol

Reines 17-Acetoxy-11β-(9-acetoxynonyl)-14,17-ethanoestra-1,3,5(10)-trien-3-ol (800 mg) wird mit 8 ml 1 normaler methanolischer Kaliumhydroxydlösung 2 Stunden bei Raumtemperatur gerührt, auf Wasser gegeben, mit 2 normaler Salzalsäure angesäuert, mit Essigester dreimal extrahiert, über Natriumsulfat getrocknet und i.Vak. eingeengt. Man erhält 670 mg rohes 14,17-Ethano-11β-(9-hydroxynonyl)-estra-1,3,5(10)-trien-3,17-diol, das in 9.3 ml Aceton gelöst, mit 2,28 ml 1 normaler Natronlauge, tropfenweise mit 0,18 ml Benzoylchlorid versetzt und 0,5 Stunden gerührt wird. Dann wird mit Essigester dreimal extrahiert, neutralgewaschen, über

Natriumsulfat getrocknet und i.Vak. eingeengt. Man erhält 830 mg rohes 3-Benzoyloxy-14,17-ethano-11β-(9-hydroxynonyl)-estra-1,3,5(10)-trien-17-ol. Eine Lösung von 830 mg 3-Benzoyloxy-14,17-ethano-11β-(9-hydroxynonyl)estra-1,3,5(10)-trien-17-ol in 10 ml Pyridin wird mit 763 mg Tosylchlorid 4,5 Stunden bei Raumtemperatur gerührt. Dann wird mit Essigester verdünnt, mit 1 normaler Salzsäure gewaschen, mit Wasser neutralgewaschen, über Natriumsulfat getrocknet, i.Vak. eingeengt und an Kieselgel mit Hexan/Essigester chromatographiert. Man erhält 480,6 mg reines 3-Benzoyloxy-14,17-ethano-11β-(9-tosyloxynonyl)-estra-1,3,5(10)-trien-17-ol als Schaum.

f) 14,17-Ethano-11β-[9-(4,4,5,5,5-pentafluorpentylsulfanyl)-nonyl]-estra-1,3,5(10)-trien-3,17-diol

Eine Lösung von 207 mg Thioessigsäure-S-(4,4,5,5,5-pentafluorpentyl)-ester in 3 ml Methanol wird 0,5 Stunden mit 60 mg Natriummethoxyd bei Raumtemperatur gerührt. Diese Lösung wird tropft man zu einer Lösung von 480 mg 3-Benzoyloxy-14,17-ethano-11β-(9-tosyloxynonyl)-estra-1,3,5(10)-trien-17-ol in 10 ml Dimethylformamid und rührt weitere 3 Stunden bei Raumtemperatur. Dann wird auf 0,1 normale Salzsäure gegeben, dreimal mit Essigester extrahiert, mit Wasser gewaschen, über Natriumsulfat getrocknet, i.Vak. eingeengt und an Kieselgel mit Hexan/Essigester chromatographiert. Man erhält 251 mg 14,17-Ethano-11β-[9-(4,4,5,5,5-pentafluorpentylsulfanyl)-nonyl]-estra-1,3,5(10)-trien-3,17-diol als Öl.

g) 9-(14,17-Ethano-3,17-dihydroxyestra-1,3,5(10)-trien-11β-yl)-nonyl-4,4,5,5,5-pentafluorpentyl-sulfoxid

Eine Lösung von 130 mg 14,17-Ethano-11β-[9-(4,4,5,5,5-pentafluorpentylsulfanyl)-nonyl]-estra-1,3,5(10)-trien-3,17-diol in 5 ml Methanol wird mit 0,2 ml Wasser und 56,2 mg Natriumperiodat 4,5 Stunden bei Raumtemperatur gerührt. Dann wird auf Wasser gegeben, mit Essigester extrahiert, mit 1 normaler Salzsäure gewaschen, mit Wasser neutralgewaschen, über Natriumsulfat getrocknet, i. Vak. eingeengt, in warmem Hexan gelöst und durch Abkühlung ausgefällt. Man erhält 84,7 mg reines 9-(14,17-Ethano-3,17-dihydroxyestra-1,3,5(10)-trien-11β-yl)-nonyl-4,4,5,5,5-pentafluorpentyl-sulfoxid.

## Patentansprüche

1. 11β-Substituierte 14,17-Ethanoestratriene der allgemeinen Formel I

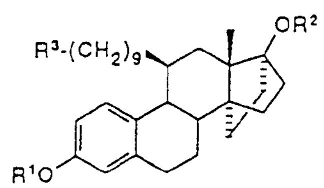

(I),

worin $R^1$ für ein Wasserstoffatom, eine $C_1$-$C_{12}$-Alkanoyl-, eine Benzoyl-, eine gerad- oder verzweigtkettige $C_1$-$C_{12}$-Alkyl-, eine $C_3$-$C_7$-Cycloalkyl- oder eine $C_4$-$C_8$-Alkylcycloalkylgruppe, $R^2$ für ein Wasserstoffatom oder eine $C_1$-$C_{12}$-Alkanoylgruppe und $R^3$ für eine Gruppierung

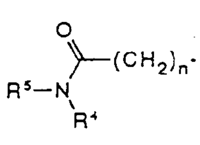 oder 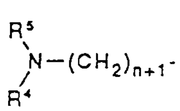 oder $R^4$-SO-

stehen, wobei n 0, 1l oder 2 ist und $R^4$ und $R^5$ unabhängig voneinander ein Wasserstoffatom oder eine geradkettige oder verzweigte $C_1$-$C_8$ -Alkylgruppe, die auch teilweise fluoriert vorliegen kann, bedeuten.

2. Verbindungen nach Anspruch 1
11-(14,17-Ethano-3,17-dihydroxyestra-1,3,5(10)-trien-11β-yl)-undecansäure-(N-methyl-N-*iso*propyl)-amid,
11-(14,17-Ethano-3,17-dihydroxyestra-1,3,5(10)-trien-11βyl)-undecansäure-(N-methyl-N-2,2,3,4,4,4,4-heptafluorbutyl)-amid,
14,17-Ethano-11β-(11-N-methyl-N-*iso*propylaminoundecyl)-estra-1,3,5(10)-trien-3,17-diol,
9-(14,17-Ethano-3,17-dihydroxyestra-1,3,5(10)-trien-11β-yl)-nonyl-4,4,5,5,5-pentafluorpentyl-sulfoxid,

10-(14,17-Ethano-3,17-dihydroxyestra-1,3,5(10)-trien-11β-yl)-decansäure-(N-methyl-N-*iso*propyl)-amid,
12-(14,17-Ethano-3,17-dihydroxyestra-1,3,5(10)-trien-11β-yl)-dodecansäure-(N-methyl-N-*iso*propyl)-amid,
11-(3-Benzoyloxy-14,17-ethano-17-hydroxyestra-1,3,5(10)-trien-11β-yl)-undecansäure-(N-methyl-N-*iso*propyl)-amid,
11-(14,17-Ethano-17-hydroxy-3-methoxyestra-1,3,5(10)-trien-11β-yl)-undecansäure-(N-methyl-N-*iso*propyl)-amid.

**3.** Verfahren zur Herstellung der Verbindungen der allgemeinen Formel I, dadurch gekennzeichnet, daß eine Verbindung der allgemeinen Formel II

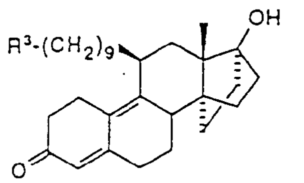

(II),

worin $R^3$ die in Formel I angegebenen Bedeutungen

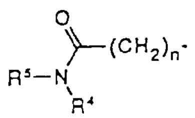 oder 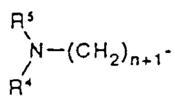 oder $R^4$-SO-

hat, mit einem Säurehalogenid/Säureanhydrid-Gemisch

$$R^{1,2}X/(R^{1,2})_2O \quad (III),$$

worin $R^{1,2}$ eine $C_1$-$C_{12}$-Alkanoylgruppe sowie X ein Chlor- oder Bromatom bedeuten, zur entsprechenden 1,3,5(10)-Trien-3-hydroxy-Verbindung der allgemeinen Formel IV

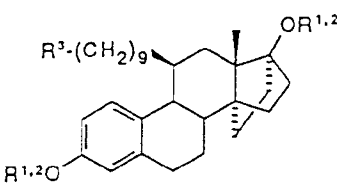

(IV),

aromatisiert und diese anschließend gegebenenfalls partiell oder vollständig verseift, gegebenenfalls partiell in 3-Position oder vollständig in 3- und 17-Position mit einem Säurehalogenid oder -anhydrid der allgemeinen Formel V

$$R^{1'}X \text{ bzw. } R^{1'}_2O \quad (V),$$

worin $R^{1'}$ eine $C_1$-$C_{12}$-Alkanoyl- oder Benzoylgruppe sowie X ein Chlor- oder Bromatom bedeuten, verestert oder partiell in 3-Position mit einem Alkylhalogenid der allgemeinen Formel VI

$$R^{1''}Y \quad (VI),$$

worin $R^{1''}$ eine gerad- oder verzweigtkettige $C_1$-$C_{12}$-Alkyl-, eine $C_3$-$C_7$-Cycloalkyl- oder eine $C_4$-$C_8$-Alkycycloalkylgruppe sowie Y ein Chlor-, Brom- oder Iodatom bedeuten, verethert und gegebenenfalls in 17-Position mit einem Säurehalogenid- oder -anhydrid der allgemeinen Formel VII

$$R^{2'}X \text{ bzw. } R^{2'}_2O \quad (VII),$$

worin $R^{2'}$ eine $C_1$-$C_{12}$-Alkanoylgruppe sowie X ein Chlor- oder Bromatom bedeuten, verestert und wenn $R^3$ für den Carbonsäureamidrest

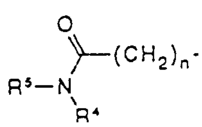

steht gewünschtenfalls dessen Ketogruppe vollständig reduziert wird.

4. Pharmazeutische Präparate, dadurch gekennzeichnet, daß sie mindestens eine Verbindung gemäß Anspruch 1 oder 2 sowie einen pharmazeutisch verträglichen Träger enthalten.

5. Verwendung der Verbindungen gemäß Anspruch 1 oder 2 zur Herstellung von Arzneimitteln.